# EUROPEAN PATENT SPECIFICATION

(11) **EP 1 991 311 B1**
(45) Date of publication and mention of the grant of the patent: **08.08.2012**
(21) Application number: 06829942.9
(22) Date of filing: 06.11.2006
(51) Int. Cl.: A61N 1/40

(54) **APPARATUS AND METHOD FOR TREATING SKIN BLEMISHES DUE TO SKIN STRIAE**
GERÄT UND VERFAHREN ZUR BEHANDLUNG VON HAUTFEHLERN DURCH HAUTSTREIFEN
APPAREIL ET PROCÉDÉ DE TRAITEMENT DES IMPERFECTIONS CUTANÉES DUES AUX VERGETURES

(30) Priority: 23.02.2006 IT FI20060047
(43) Date of publication of application: 19.11.2008
(73) Proprietor: Busoni, Maurizio, 50144 Firenze (IT)
(72) Inventor: Busoni, Maurizio, 50144 Firenze (IT)
(74) Representative: Gervasi, Gemma
(86) International application number: PCT/EP2006/068133
(87) International publication number: WO 2007/096009

(56) References cited:
- WO-A-98/05286
- WO-A-03/005921
- WO-A-03/018117
- WO-A-03/079916
- WO-A-2005/112807
- JP-A- 3 162 870
- US-A1- 2006 036 300

## Description

### Field of the invention

The present invention relates to the field of apparatuses and methods for the treatment of skin blemishes due to skin striae.

### State of the art

Striae are generally the result of rapid dimensional alterations of the skin, subject to higher tension due for example to weight increase or stretching of the abdomen during pregnancy and enlargement of the breast during breast-feeding. Indeed, such increases of weight cause the skin to be subject to proper tears which manifest themselves as striae, proper skin lesions which appear as narrow, elongated grooves, within which the skin becomes progressively more opaque, rigid and devitalised, gradually increasing ever more evidently the difference with the surrounding tissue.

Cosmetic treatments adapted to remedy the formation of striae comprise various type of interventions in order to obtain evident results. These include non-invasive techniques, such as:
- the application of cosmetic products which act on the tissue surrounding the skin striae aiming at obtaining a better elasticity of the skin and/or an improvement of skin microcirculation;
- the application of cosmetic peeling products which act by reducing the thickness of the stratum corneum;
- the carrying of cosmetic products and medications by supplying direct current (ionophoresis), which allows an effect similar to that previously shown, with higher potentials given the probable higher percentage of product capable of crossing the physiological barriers of our skin;
- abrasive treatments with mechanical tools so as to remove part of the stratum corneum cells coating the external part of the striae, making them thicker, harder and more rigid with respect to the surrounding skin tissue;
- abrasive treatment with a jet of corundum crystals or other abrasive material addressed directly to the striae, thus removing part of the stratum corneum;
- the application of surface cosmetics so as to allow a concealing effect of the striae and of the visual and aesthetic consequences.

All the previously shown techniques have a common limit due to the nature of the blemish itself. Being skin striae a degeneration of tissues which culminates in a surface sclerosis, it is perfectly understandable how the application of cosmetic products aimed at improving skin elasticity and vascularisation is hindered precisely by the higher sclerosis of the tissue and the consequent further difficulty in crossing precisely the barrier formed by the stratum corneum, whose higher compactness and density correspond to a proportional impediment and hindering precisely of the uptake of cosmetic product, also when potentially supported by the administration of direct current (ionophoresis) finalised precisely to "push" the cosmetic itself in further depth.

Instead, the application of abrasive or peeling treatments, either cosmetic products or applications using mechanical tools or a jet of corundum crystals or other material or abrasive product, actually allow to reduce the thickness of the stratum corneum, although this generates the risk of excessive abrasion, particularly with the adoption of corundum crystals, notwithstanding that the reduction of the thickness of the stratum corneum represents a temporary result, as the stratum corneum is physiologically intended to become progressively thicker in time, given natural cellular denucleation.

Finally, the application of products which allow to conceal the striae allow a resolutely temporary and only token, never structural, result.

Therefore, all the previously illustrated methods have a direct action either on the tissue surrounding skin striae or the stratum corneum which covers them, allowing to either incompletely or temporarily attenuate the blemishes deriving from the formation of the striae.

It is therefore evident the interest to be able to eliminate the aforesaid limits availing of an apparatus which allows to successfully face the aesthetic problem of striae. Three aspects which characterise blemishes due to skin striae:
- improving elasticity and microcirculation functionality localised in the tissue surrounding striae;
- increasing temperature in the range of tissue surrounding the skin striae to reach a temperature of approximately thirty-nine degrees centigrade, i.e. two degrees over physiological temperature;
- contributing to increasing metabolic activity by combining the action intended to increase vascularisation with the increase of intracutaneous temperature.

The document WO03/079916 which may be considered as constituting the closest prior art, discloses an apparatus comprising:
- a bell hand piece comprising an electrode;
- a vacuum generating device;
- an electrical current generating device;
- a logical unit intended to manage and adjust the two systems described above.

This document, however, does not disclose:
- a reference handpiece
- an electrode coated with dielectric material
- a temperature raise up to approximately 39 degrees

### Summary

The present invention refers to an apparatus for the treatment of skin blemishes capable of allowing, at the same time, a vascularisation of the skin surface to be treated and an increase of temperature within the skin.

### Brief description of the drawings

Figure 1 schematically shows the apparatus according to the invention;
Figure 2 shows a particular embodiment of the bell handpiece, shown in Fig. 1 with the respective electrode;

### Detailed description of the invention

The present invention allows to solve the aforesaid problems thanks to an apparatus which allows to exploit the combined action of surface vascularisation and the increased temperature within the skin allowing to raise and progressively fill the tissue surrounding the striae and thus allowing a progressive stabilisation of the result, ensured by the vital cycle of the new cells deriving from increased mitosis.

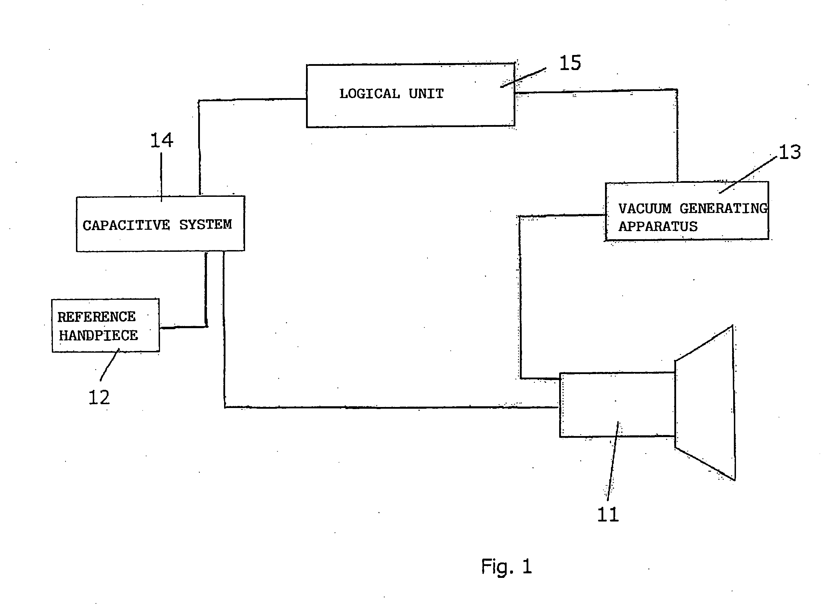

As shown in Fig. 1, apparatus 10 according to the invention comprises:
- a bell handpiece 11;
- a reference handpiece 12;
- a vacuum generating apparatus 13;
- an electrical current generating device 14;
- a logical unit 15 intended to manage and adjust the two systems described above;
- an electrode 16 (contained in active handpiece 11).

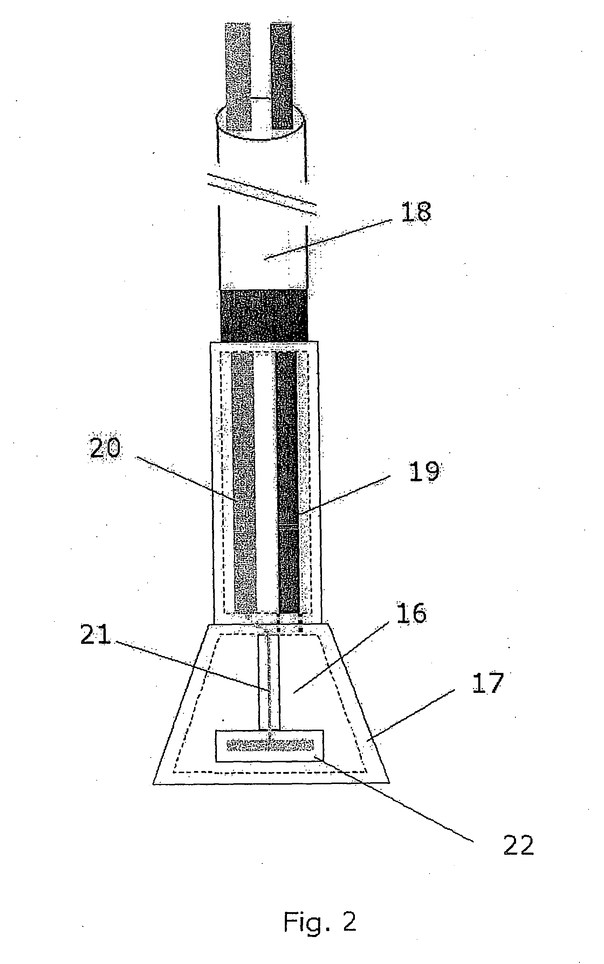

As shown in figure 2, (active) bell handpiece 11 consists of a hollow, essentially bell-shaped structure 17, within which vacuum may be created, connected to a pneumatic cable 19, which connects the bell to vacuum creation system 13, and an electrical wire 20 which connects electrode 16 (see below) to the electrical current generating device 14, preferably said pneumatic cable 19 and said electrical wire 20 are accommodated in an appropriate hollow conduit 18.

Bell structure 17 further contains an electrode 16 which allows a capacitive coupling effect, i.e. an overheating of what comes into contact with it.

Said electrode 16 is arranged in position so as to allow contact with the skin to be treated, preferably in the middle of bell 17.

Vacuum generating apparatus 13 comprises a vacuum pump preferably connected to a reservoir which is also preferably connected to a sensor for reading and adjusting the real vacuum level within the bell (and therefore actually applied to the part to be treated) which acts on a battery of solenoid valves, which determine a loss of vacuum proportional to the requirements of use.

Air filters are normally arranged downstream of said battery of solenoid valves and therefore downstream of the connection, by means of pneumatic cable 19, to the active handpiece 11 to be applied directly to the skin on the points to be treated

The electrical current generating device consists of an electrical circuit comprising an alternating current power supply connected to electrode 16 by means of electrical wire 20 and to a second electrode, consisting of reference handpiece 12, by means of a second electrical wire 22.

The system is capable of generating a high-frequency alternating current which is applied to the skin through electrode 16 while the treated user grips reference handpiece 12.

Said reference handpiece 12 is normally a rod, which may be ergonomically shaped, consisting of current conducting material.

Electrode 16, connected to the electrical current generating device 14 in the way described above, presents one internal conductive part 21, coated with a dielectric material 22 intended to come into contact with the skin tissue.

As understandable, electrode 16 and reference handpiece 12 act as plates of a capacitor in which the treated tissues, as the isolating coating of electrode 22, form the dielectric. When the current is supplied, rapidly alternating charges which determine within the treated tissue an electrical field which equally rapidly changes direction so as to generate heat, are established on the insulation-coated electrode. The temperature of the derma is thereby raised to approximately 39 degrees celsuis.

The logical unit according to the invention consists of a printed circuit made so as to accommodate a microprocessor capable of managing all power parts, i.e. the vacuum generating part and the electrical current generating part; it is provided with a screen or display allowing to view all the functions of the apparatus, a keyboard for managing the apparatus and all its functions and a timer thanks to which the apparatus automatically switches off the power parts at the end of a predetermined time.

According to the invention, bell handpiece 11 may have various dimensions, in relation to the different extensions and stretching of the striae or to practical and functional aspects, and so may the electrodes intended to generate the capacitive (i.e. thermal) effect may be of different dimensions and coated with dielectrics of various nature.

As mentioned, according to a particular embodiment of the invention, the apparatus allows to adjust both the level of vacuum applied to the part to be treated and the capacitive energy transfer level, thus in fact adjusting also the level of overheating of the derma and hypodermis.

Preferably, the apparatus will also be provided with a timer adapted to switch it off automatically after a certain period of time.

If required, the apparatus may contemplate a selector which allows to activate the vacuum, the thermal effect or the two functions at the same time and/or possible selectors which allow to set vacuum levels which alternate higher or lower values, so as to determine a proper pumping action in relation to skin microcirculation.

The apparatus will obviously comprise the necessary circuitry for connecting the various aforesaid parts and for connecting to the power supply (mains, batteries, etc.).

Normally, vacuum generating apparatus 13, electrical current generating apparatus 14 and logical unit 15 will be accommodated in an appropriate structure from which the power wire of the apparatus, the pneumatic cable 19 and the electrical wire 20 (possibly contained in hollow 18) exit and respectively connect the vacuum generating system and the capacitive system to the handpiece and the respective electrode and electrical wire 22 connected to reference handpiece 12.

The apparatus according to the invention will then be provided with the necessary systems for displaying all set and/or selected values for the treatment and therefore the required supplies.

Furthermore, if required, the apparatus according to the invention may also be provided with appropriate complementary devices, intrinsically known, which allow to improve the effects of the cosmetic treatment.

Such possible complementary devices may include, for example:
- tools provided with abrasive surface capable of performing an effective peeling, connected to the vacuum pump by means of a pneumatic cable possibly provided with a filter intended to withhold impurities;
- handpieces provided with a micro motor capable of turning brushes of bristles or other materials applied to them to perform an effective peeling, said handpieces being connected to a current generator present within the machine body, by means of a two-pole electrical wire;
- handpieces connected to a current generator so as to supply various types of pulses, which include by way of non-comprehensive example, direct current, stimulation by means of square waves, sinusoidal waves, triangular waves, stimulation by means of half-waves, and by means of a two-pole electrical wire; said handpieces allow to exert a pumping action in relation to skin blood and lymph microcirculation;
- plates of various shapes and dimensions intended for hot/cold generation either by the possible adoption of resistors or infrared generators for heat or Peltier cells for combined hot and cold management or for the generation of cold alone connected to a current generator arranged in the machine body by means of a two-pole wire;
- handpieces or plates of various shape, intended to heat the treated tissue, favouring a better reactivation of metabolism connected to a radiofrequency generator by means of a suitable wire, preferably shielded of the RG58 or similar type;
- handpieces of plates of various shape, aimed at both delivering synergetic treatment active substances and of exerting a pumping action in relation to skin blood and lymph microcirculation connected to an ultrasound generator by means of a suitable wire, preferably shielded of the RG58 or similar type;
- interchangeable handpieces of various shape connected to the vacuum pump existing in the apparatus with a pneumatic cable, possibly provided with a filter for withholding impurities, intended to exert a vacuum effect on the skin, so as to favour an attraction of blood and lymph into the treated area.

The use of the apparatus according to the invention is apparent.

After setting the values needed for the treatment and having activated the apparatus, the tool is passed directly over the striae, repeatedly shifting it along the same until the tissue appears red. From this moment onwards, two actions will occur at the same time, i.e. the increase of skin tissue vascularisation and of the intracutaneous temperature which allow to obtain the following advantages:
- increase of blood flow with consequent higher quantity of oxygen, directly within the tissue underneath the striae. The formation of striae causes a higher thickening of the stratum corneum which proportionally to thickness hinders the physiological skin transpiration, by means of which a significant contribution of oxygen should occurs destined to cellular nutrition, in this case drastically reduced;
- increase of temperature within the tissue to reach approximately thirty-nine degrees Celsuis, temperature thanks to which, according to Van't Hoff's law, cellular mitosis phenomena may be increased up to three-fold. The combination of raised temperature with a higher blood flow remains essential, without which there would probably not be the amount of oxygen needed to increase mitosis;

- filling of tissue underneath the striae thanks both to the higher volume of blood and lymph vessels present within the treated skin tissue, and to the presence of new cells deriving from increased mitosis;
- volumetric increase of lymphatic vessels allows increase of fluids within the treated tissue, allowing a higher skin moisturisation;
- the effect of the higher cellular reproduction, of the higher vascularisation and moisturisation of the tissue progressively allows to increase elasticity of the tissue itself contributing to reducing the hardness and stiffness typical of tissue effected by striae;
- the repeated application of the treatment allows a stabilisation of the result for a reasonably prolonged time, thanks to the higher stabilisation of the superior vascularisation of the tissue, the consequent oxygenation and skin moisturisation and a progressively prominent cellular reproduction;
- the combination of the thermal effect further allows a higher fluidity of lymphatic microcirculation, which proportionally increases the faculty of reabsorbing toxins and waste present within the intercellular space, generally abundant in the tissue effected by striae, as demonstrated by the complexion of the striae themselves, usually much more opaque than the surrounding tissue,

It is important to observe how the apparatus is extremely simple to use and does not require the intervention of particularly specialised and/or trained personnel.

## Claims

1. An apparatus for the treatment of skin blemishes capable of allowing, at the same time, a vascularisation of the skin surface to be treated and raising the temperature of the derma to which it is applied up to approximately 39 degrees , said apparatus comprising:
- a bell handpiece (11) comprising an electrode (16)
- a reference handpiece (12);
- a vacuum generating device (13);
- an electrical current generating device (14);
- a logical unit (15) intended to manage and adjust the two systems described above;
wherein said electrode (16) presents an internal conductive part (21) coated with dielectric material (22) intended to come into contact with the skin tissue,
wherein said bell handpiece (11) consists of an essentially bell-shaped hollow structure (17), within which vacuum may be formed, connected to a pneumatic cable (1), which connects the bell to the vacuum generating system (13), and an electrical wire (20) which connects the electrode (16) to the electrical current generating device (14);
wherein the electrical current generating device consists of an electrical circuit comprising an alternating current power supply connected to electrode (16) by means of electrical wire (20) and to a second electrode, consisting of reference handpiece (12), by means of a second electrical wire.

2. An apparatus according to claim 1, wherein said pneumatic cable (19) and electrical wire (20) are accommodated in an appropriate hollow conduit (18).

3. An apparatus according to claim 2, wherein said vacuum generating device (13) comprises a vacuum pump connected to a reservoir which is further preferably connected to a sensor for reading and adjusting the real vacuum level existing in the bell actually applied to the part to be treated, which acts on a battery of solenoid valves, which determine a loss of vacuum proportional to the requirements of use.

4. An apparatus according to claim 3, wherein said reference handpiece (12) is a rod, which may be ergonomically shaped, consisting of current conducting material.

5. An apparatus according to claims 1-4, wherein said logical unit is a printed circuit made so as to accommodate a microprocessor capable of managing all power parts, i.e. the vacuum generating part and the electrical current generating part and is provided with a display for viewing all the functions of the apparatus, and a keyboard for managing the apparatus and all its functions.

6. An apparatus according to claim 5, wherein said logical unit is provided with a timer adapted to automatically switch it off after a certain period of time.

7. An apparatus according to claims 1 - 6 provided with a selector capable of allowing to activate the vacuum, the thermal effect or the two functions at the same time and/or possible selectors which allow to set vacuum levels which alternate higher or lower values, so as to determine a proper pumping action in relation to skin microcirculation.

8. An apparatus according to claims 1-7, wherein the vacuum generating device (13), the electrical current generating device (14) and logical unit (15) are accommodated in an appropriate structure from which the power wire of the apparatus, the pneumatic cable (19) and the electrical wire (20), possibly contained in the wire (18), exit and respectively connect the vacuum generating system and the electrical current generating device to the handpiece (11) and the respective electrode and to the electrical wire (22) connected to the reference handpiece (12).

9. A method for the cosmetic treatment of skin blemishes due to striae using the apparatus of any of the previous claims, wherein:
- the values required for treatment are set and the apparatus is activated;
- the apparatus is passed directly over the skin blemish, repeatedly shifting it along the same until the tissue becomes red;
- application is repeated for the time deemed appropriate.

## Patentansprüche

1. Gerät zur Behandlung von Hautflecken, welches in der Lage ist, gleichzeitig eine Vaskularisierung der zu behandelnden Hautoberfläche und einen Anstieg der Temperatur der Hautstelle, auf die es angewendet wird, bis auf annähernd 39 Grad zu ermöglichen, wobei das genannte Gerät umfasst:
- ein glockenförmiges Handstück (11) welches eine Elektrode (16) umfasst;
- ein Referenz-Handstück (12);
- eine Unterdruck erzeugende Vorrichtung (13);
- eine Vorrichtung zur Erzeugung von elektrischem Strom (14);
- eine Logikeinheit (15), welche dazu dient, die beiden weiter oben beschriebenen Systeme zu steuern und einzustellen,
wobei die genannte Elektrode (16) ein inneres leitendes Teil (21) aufweist, welches mit einem dielektrischen Material (22) beschichtet ist, das mit dem Hautgewebe in Kontakt gebracht werden soll,
wobei das genannte glockenförmige Handstück (11) aus einem im Wesentlichen glockenförmigen Hohlkörper (17) besteht, in welchem Unterdruck aufgebaut werden kann und an den eine pneumatische Leitung (19), welche die Glocke mit dem Unterdruckerzeugungssystem (13) verbindet, und ein elektrisches Kabel (20), welches die Elektrode (16) mit der Vorrichtung zur Erzeugung von elektrischem Strom (14) verbindet, angeschlossen sind,
wobei die Vorrichtung zur Erzeugung von elektrischem Strom aus einem Stromkreis besteht, welcher eine Stromversorgung mit Wechselstrom umfasst, welche über ein elektrisches Kabel (20) mit der Elektrode (16) und mittels eines zweiten elektrischen Kabels an eine zweite Elektrode angeschlossen ist, welche zum Bezugshandstück (12) gehört.

2. Gerät nach Anspruch 1, bei welchem die genannte pneumatische Leitung (19) und das elektrische Kabel (20) in einer geeigneten hohlen Führung (18) untergebracht sind.

3. Gerät nach Anspruch 2, bei welchem die genannte Vakuum erzeugende Vorrichtung (13) eine Vakuumpumpe umfasst, welche an einen Vorratsbehälter angeschlossen ist, an den außerdem vorzugsweise ein Sensor zum Zweck der Anzeige und der Einstellung des tatsächlichen Unterdruckes angeschlossen ist, der an den zu behandelnden Teil tatsächlich angelegt wird, wobei dieser Sensor auf eine Reihe von Magnetventilen wirkt, über welche eine Abnahme des Unterdrucks je nach den Anforderungen des Einsatzes festgelegt wird.

4. Gerät nach Anspruch 3, bei welchem das genannte Referenz-Handstück (12) ein Stab ist, welcher ergonomisch gestaltet sein kann und der aus einem stromleitenden Material besteht.

5. Gerät nach den Ansprüchen 1-4, bei welchem die genannte Logikeinheit eine gedruckte Schaltung ist, welche so ausgelegt ist, dass sie einen Mikroprozessor enthält, welcher imstande ist, alle Baugruppen mit elektrischer Leistung, d. h. die das Vakuum erzeugende Baugruppe und die stromerzeugende Baugruppe, zu steuern, und welche eine Anzeigevorrichtung aufweist, um all die Funktionen des Gerätes zur Anzeige zu bringen, sowie eine Tastatur zur Steuerung des Gerätes und all seiner Funktionen.

6. Gerät nach Anspruch 5, bei welchem die genannte Logikeinheit mit einem Zeitgeber ausgestattet ist, der so ausgelegt ist, dass er das Gerät nach einer gewissen Zeitspanne abschaltet.

7. Gerät nach den Ansprüchen 1-6, welches mit einem Wahlschalter ausgestattet ist, welcher ermöglicht, den Unterdruck anzulegen, die thermische Wirkung einzuleiten oder die beiden Funktionen gleichzeitig zu aktivieren, und/oder mit möglichen Wahlschaltern, welche ermöglichen, Unterdruck mit wechselndem höheren oder niedrigerem Wert einzustellen, so dass eine passende Pumpwirkung in Bezug auf die Mikrozirkulation der Haut festgelegt wird.

8. Gerät nach den Ansprüchen 1-7, bei welchem die den Unterdruck erzeugende Vorrichtung (13), die den elektrischen Strom erzeugende Vorrichtung (14) und die logische Einheit (15) in einem geeigneten Gehäuse untergebracht sind, von welchem die Stromversorgungsleitung für das Gerät, die pneumatische Leitung (19) und das elektrische Kabel (20), welches möglicherweise in der Führung (18) untergebracht ist, abgehen bzw. das System zur Erzeugung des Vakuums und die Vorrichtung zur Erzeugung des elektrischen Stroms mit dem Handstück (11) und der zugehörigen Elektrode und mit dem elektrischen Kabel (22), das an das Bezugshandstück (12) angeschlossen ist, verbinden.

9. Verfahren zur kosmetischen Behandlung von durch Hautstriemen bedingten Hautflecken unter Verwendung des genannten Gerätes nach irgend einem der vorgenannten Ansprüche, wobei:
- die für die Behandlung erforderlichen Werte eingestellt werden und das Gerät in Betrieb genommen wird;
- das Gerät direkt über den Hautfleck geführt wird und wiederholt längs desselben hin und her geschoben wird, bis das Gewebe rot wird;
- die Anwendung wiederholt wird über eine Zeitspanne, welche für geeignet erachtet wird.

## Revendications

1. Appareil de traitement des imperfections cutanées apte à permettre, en même temps, une vascularisation de la surface de la peau à traiter et l'augmentation de la température du derma auquel il est appliqué jusqu'à environ 39 degrés, ledit appareil comprenant:
une pièce à main en forme de cloche (11) comprenant une électrode (16);
une pièce à main de référence (12);
un dispositif générateur de vide (13);
un dispositif générateur de courant électrique (14);
une unité logique (15) destinée à gérer et à ajuster les deux systèmes décrits ci-dessus;
où ladite électrode (16) présente une partie conductrice interne (21) revêtue de matériau diélectrique (22) destinée à venir en contact avec le tissu de la peau, où ladite pièce à main formant cloche (11) est constituée d'une structure creuse essentiellement en forme de cloche (17), dans laquelle un vide peut être formé, reliée à un câble pneumatique (1), qui relie la cloche au système générateur de vide (13), et un fil électrique (20) qui relie l'électrode (16) au dispositif générateur de courant électrique (14);
où le dispositif générateur de courant électrique est constitué d'un circuit électrique comprenant une alimentation en courant alternatif connectée à l'électrode (16) par un fil électrique (20) et à une deuxième électrode, constituée de la pièce à main de référence (12), au moyen d'un deuxième fil électrique.

2. Appareil selon la revendication 1, où ledit câble pneumatique (19) et le fil électrique (20) sont logés dans un conduit creux approprié (18).

3. Appareil selon la revendication 2, où ledit dispositif générateur de vide (13) comprend une pompe de vide reliée à un réservoir qui est en outre connecté de préférence à un capteur pour la lecture, et l'ajustement du niveau de vide réel existant dans la cloche actuellement appliquée à la partie à traiter, qui agit sur une batterie de vannes à solénoïde, qui déterminent une perte de vide proportionnelle aux exigences d'utilisation.

4. Appareil selon la revendication 3, où ladite pièce à main de référence (12) est une tige, qui peut avoir une forme ergonomique, constituée d'un matériau conducteur de courant.

5. Appareil selon les revendications 1 à 4, où ladite unité logique est un circuit imprimé réalisé pour recevoir un microprocesseur apte à gérer toutes les parties de puissance, c'est-à-dire la partie générateur de vide et la partie générateur de courant électrique et présente un affichage pour voir toutes les fonctions de l'appareil, et un clavier pour gérer l'appareil et toutes ses fonctions.

6. Appareil selon la revendication 5, où ladite unité logique présente une horloge apte à la mettre automatique hors service après une certaine période de temps.

7. Appareil selon les revendications 1 à 6, muni d'un sélecteur apte à permettre l'activation du vide, l'effet thermique ou les deux fonctions en même temps et/ou des sélecteurs possibles qui permettent le réglage des niveaux de vide qui alternent entre des valeurs plus élevées ou plus basses de manière à déterminer une action de pompage appropriée relativement à la microcirculation cutanée.

8. Appareil selon les revendications 1 à 7, où le dispositif générateur de vide (13), le dispositif générateur de courant électrique (14) et l'unité logique (15) sont reçus dans une structure appropriée de laquelle sortent le fil de puissance de l'appareil, le câble pneumatique (19) et le fil électrique (20), se trouvant éventuellement dans le fil (18), et relient respectivement le système générateur de vide et le dispositif générateur de courant électrique à la pièce à main (11) et à l'électrode respective et au fil électrique (22) connecté à la pièce à main de référence (12).

9. Procédé pour le traitement cosmétique des imperfections cutanées dues à des vergetures en utilisant l'appareil selon l'une quelconque des revendications précédentes, où:
- les valeurs requises pour le traitement sont réglées, et l'appareil est activé;
- on fait passer l'appareil directement sur l'imperfection cutanée, en le faisant passer plusieurs fois le long de celle-ci jusqu'à ce que le tissu devienne rouge;
- l'application est répétée pendant le temps estimé comme étant approprié.
